# EUROPEAN PATENT SPECIFICATION

(11) **EP 1 443 917 B1**
(45) Date of publication and mention of the grant of the patent: **29.03.2006**
(21) Application number: 02775597.4
(22) Date of filing: 01.11.2002
(51) Int. Cl.: A61K 31/18, A61K 9/20, A61K 9/48, A61K 9/28, A61P 13/08

(54) **TAMSULOSIN TABLETS**
TAMSULOSIN TABLETTEN
COMPRIMES DE TAMSULOSIN

(30) Priority: 07.11.2001 US 331055 P
(43) Date of publication of application: 11.08.2004
(73) Proprietor: Synthon B.V., 6545 CM Nijmegen (NL)
(72) Inventor: PLATTEEUW, Johannes, J., NL-5215 BL 'S-Hertogenbosh (NL); VAN DALEN, Frans, NL-6524 CK Nijmegen (NL); LEMMENS, Jacobus, M., NL-6585 KM Mook (NL)
(74) Representative: Duxbury, Stephen
(86) International application number: PCT/NL2002/000695
(87) International publication number: WO 2003/039530

(56) References cited:
- WO-A-01/74390
- WO-A-98/37872
- WO-A-02/067651
- US-A- 4 772 475

## Description

### BACKGROUND OF THE INVENTION

The present invention relates to pharmaceutical tablets that contain tamsulosin, to unit dosage forms made therefrom, and to processes for making the same.

Tamsulosin is the common name for 5-[2-[[2-(2-ethoxyphenoxy)ethyl]amino] propyl]-2-methoxy-benzenesulfonamide of the formula (1). 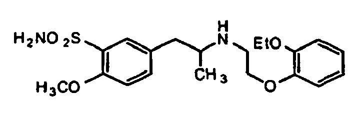 It is disclosed in EP 34432 and US 4731478 as a pharmaceutically active substance having alpha-adrenergic blocking activity that is useful for treatment of cardiac insufficiencies and benign prostatic hyperplasia.

The enantiomer (R)-tamsulosin as a hydrochloride salt is marketed in various countries including the United States for treatment of symptoms of benign prostatic hyperplasia (also known as BPH) such as urinary volume and frequency problems. The approved drug product is a capsule dosage form for oral administration that comprises only 0.4 mg of the tamsulosin hydrochloride. The capsule provides controlled release of the tamsulosin and is a once daily dosage form, although two capsules can be used if needed; i.e. a single daily administration of 0.8mg.

US 4,772,475 (EP 194838, EP 533297) discloses controlled-release pharmaceutical dosage forms comprising multiple granulate units containing tamsulosin, microcrystalline cellulose and a release control agent. The granulate gradually releases tamsulosin from the granulate matrix. It is believed that the inventive idea and compositions disclosed in US 4,772,475 cover the commercially marketed capsule product.

The disclosed process for producing the granulate units comprises granulating a mixture of tamsulosin, an unit-forming inert material such as microcrystalline cellulose and a release controlling agent comprising water and/or an aqueous emulsion, suspension or gel of a water-insoluble macromolecular substance or a solution of said macromolecular substance in an aqueous organic solvent. The macromolecular substance is preferably selected from a range of acrylate polymers, commercially sold under brand name Eudragit®. The release controlling agent serves essentially also as a binder in the granulation process. The resulting granulate may be used for making final dosage forms, capsules as well as tablets.

Example 1 of US 4,772,475 illustrates the process. After sufficiently mixing 5 g tamsulosin HCl and 470 g microcrystalline cellulose, a mixture of 83.3 g (25 g as solid component) of Eudragit L 30 D-55 and 500 g of water was added thereto and the resultant mixture was granulated by a high-speed mixer. The granules obtained were spheres having particle sizes of 0.1 to 1.5 mm, mainly 0.2 to 1.0 mm. The apparently required use of a liquid, water in this case, in forming the granulates is not surprising given the low amount of active being mixed relative to the amount of excipients. However, the water has to be removed after granulating, making the process very energy and time-consuming. It is especially difficult is to remove water from the interior of the solid granules. Also, the enhanced temperatures necessary for proper drying can lead to the formation of undesired side-products. The impurities may also be formed by hydrolysis or by the action of oxygen dissolved in the water. The same is essentially true if a solvent other than water is used in the granulation process.

US 4,772,475 also recites the formation of a tablet as a comparison product to the capsules. The tablets comprise tamsulosin hydrochloride, lactose, starch /com starch and starch paste. The presence of starch paste indicates that the tablets were also prepared by an aqueous process, i.e. by the same process as used for the granules, rising the potential for the same disadvantages.

It would be beneficial to be able to make a tamsulosin pharmaceutical tablet or dosage form that did not use water or a liquid in mixing. While dry methods in general are well known, including direct compression and dry granulation techniques, such methods are not thought to be suitable for dosage forms where the amount of active is less than 2% by weight, and not acceptable when less than 1% is used.

### SUMMARY OF THE INVENTION

It has now been discovered that a tablet containing small amounts of tamsulosin active can be made with good content uniformity without the need to use water or a solvent. Accordingly one aspect of the invention relates to a pharmaceutical dry made tablet comprising 0.1 to 1.5 % tamsulosin active material and at least one pharmaceutically acceptable excipient. Surprisingly, tamsulosin is an easily dispersible active and thus is amenable to a dry process even though small amounts are used. Because of this feature, a batch of pharmaceutical tablets, comprising a plurality of dry made tablets containing between 0.1 and 1.5% of a tamsulosin active material and at least one pharmaceutically acceptable excipient, can be made wherein the variation in the amount of tamsulosin active material between tablets is not more than 10%, preferaby not more than 5%, more preferably not more than 2.5%.

Another aspect of the invention relates to a unit dosage form for treating or ameliorating the conditions of benign prostatic hyperplasia comprising an effective amount of one or more of the above dry made tablets. Advantageously, the tablet composition(s) are essentially bioequivalent to the marketed capsule forms. In one embodiment, several tablets, or mini-tablets, are placed into a capsule for easier administration.

A further aspect of the invention relates to a process for making tamsulosin tablets which comprises forming a tablet without the aid of a liquid wherein said tablet contains 0.1 to 1.5 % tamsulosin active material and at least one pharmaceutically acceptable excipient. Preferably the process comprises blending a tamsulosin active material with a pharmaceutically acceptable excipient to form a first blend; additionally blending the first blend with one or more additional excipients in one or more steps to form a precompression blend; and compressing the precompression blend into tablets. Milling is carried out in certain embodiments after the first blending or after an optional compaction step as part of a dry granulation technique.

### DETAILED DESCRIPTION OF THE INVENTION

The present invention relates to pharmaceutical dry made tablets containing low amounts tamsulosin active material and to processes for making the same. As used herein a "tamsulosin active material" is any tamsulosin moiety-containing substance that exhibits pharmaceutical activity and specifically includes tamsulosin free base, pharmaceutically acceptable salts thereof, especially pharmaceutically acceptable acid addition salts thereof, and mixtures or combinations of two more of any such tamsulosin substances. Examples of useful tamsulosin pharmaceutically acceptable salts include tamsulosin hydrochloride, tamsulosin bromide, tamsulosin methane sulfonate, tamsulosin tosylate, tamsulosin besylate, tamsulosin acetate, tamsulosin maleate, tamsulosin tartrate, and tamsulosin citrate. Typically the hydrochloride salt is used. The tamsulosin present in the tamsulosin active material is normally the (R)-enantiomer of tamsulosin but the (S)-enantiomer as well as mixtures of the two in various proportions including equimolar or racemic mixtures are also included within the meaning of a tamsulosin active material.

The tamsulosin active material is generally used in the form of a powder; i.e. fine particles. The particles can have a variety of particle size distributions but preferably at least 90% of the particles are 100 µm (microns) or less in particle size, more preferably 50 µm (microns) or less, still more preferably 20 µm (microns) or less, and most preferably 10 µm (microns) or less. In general a smaller particle size assists in forming a homogeneous blend and thus in meeting content uniformity goals. The particles of tamsulosin active material are usually identifiable in the dry made tablet if inspected by suitable means such as scanning electron microscopy. Accordingly, the above size distribution applies to the tamsulosin active material in all stages of the tabletting process including as a free powder, as a blend with one or more excipients, and in the tablet itself.

The amount of tamsulosin active material present in the tablet is relatively low, namely 0.1 to 1.5 %. As used herein all percentages refer to weight percent based on the entire weight of the tablet without taking into account any coating thereon, unless otherwise indicated. Typically the amount of tamsulosin active material is within the range of 0.1 to 1.2 %, more typically 0.2 to 1.0 %, preferably 0.2 to 0.8 %, and in many embodiments 0.3 to 0.6 %. In absolute terms, the amount of tamsulosin active material is generally within the range of 0.1 to 1.2 mg, typically 0.3 to 1.2 mg, and preferably 0.3 to 0.8 mg, expressed in terms of the amount of free base. For example, 0.4 mg of tamsulosin HCl is a preferred amount of tamsulosin active material which corresponds to 0.367 mg of tamsulosin free base. A preferred embodiment of the present invention contains 0.4 mg +/- 0.04 of tamsulosin HCl or multiples thereof; i.e. 0.8 mg.

The tablets of the present invention further contain at least one pharmaceutically acceptable excipient. An "excipient" as used herein means any pharmaceutically acceptable inactive component of the composition. As is well known in the art, excipients include diluents, binders, lubricants, disintegrants, release modifying agents, colorants, preservatives, pH-adjusters, etc. The excipients are selected based on the desired physical aspects of the final form, the desired release rate of the active substance from the composition after its ingestion, and the ease/cost of manufacture. In general the tablets of the present invention contain at least one of a pharmaceutically acceptable polymer, a carbohydrate or a compressible diluent. These categories are not mutually exclusive and in fact some compounds will qualify under at least two of the categories; i.e. microcrystalline cellulose is both a polymer and a compressible diluent. The polymers include a wide variety of polymeric substances including matrix forming polymers, erodible polymers, water-soluble polymers, water-insoluble polymers, water swellable polymers, pH sensitive polymers and pH-insensitive polymers among others. Specific examples of suitable polymeric materials include celluloses such as microcrystalline cellulose, cellulose acetate, ethyl cellulose, hydroxypropyl cellulose and hydroxypropylmethyl cellulose (HPMC); acrylates, methacrylates and co-polymers thereof with various co-monomers; and polyvinyl pyrrolidones. Carbohydrates include lactose, mannitol, maltodextrin, cyclodextrins, dextrates, and dextrin. Compressible diluents include any pharmaceutically acceptable diluent that is suitable for direct compression especially calcium phosphates such as calcium hydrogen phosphate dihydrate and anhydrate forms.

Generally the tablet is formulated with a diluent or carrier. Microcrystalline cellulose is a suitable inert carrier for formulating tamsulosin into tablets by the process of the invention. A suitable brand of microcrystalline cellulose is Avicel™ PH 102. Alternatively, anhydrous lactose, starch, anhydrous calcium hydrogen-phosphate, mannitol or maltodextrines may be used as suitable carriers. The inert carrier may also be formed from a mixture of two or more components. A preferred example of a combination is a mixture of anhydrous lactose and calcium hydrogen phosphate.

The tablet may optionally contain a release controlling agent in addition to or instead of the diluent /carrier. Suitable release controlling agents for use in tablet compositions of the invention may comprise any suitable solid-state acrylate/methacrylate polymer/copolymer generally approved for pharmaceutical purpose such as various types of Eudragit or Carbopol. Alternatively, it may comprise a cellulose derivative such as various grades of ethyl cellulose, hydroxypropyl cellulose or, preferably, HPMC (Methocel™) or a polyvinylpyrrolidone (Kollidon™). Water and aqueous solutions are specifically excluded as release controlling agents. There is no particular restriction as far as the amount of the release-controlling agent is concerned. It should however be able, together with tamsulosin component and the inert carrier, to be processed into the tablet form by means of direct compression or dry granulation, and to provide tablets with desired physical parameters. The nature and grade of both the release control agent and the inert carrier, as well as the mutual ratio of both ingredients in the tablet composition may have decisive influence on the final release rate. For instance, replacing the water-insoluble microcrystalline cellulose by a water-soluble lactose may result by faster gel formation when contacted with the body fluids, and it therefore provides more delayed release profile. On the other hand, various viscosity grades of HPMC allow for very similar dissolution profiles. For water-soluble or very low concentration drug, diffusion contributes predominantly to the overall dissolution and is proportional to polymer concentration at any (relatively high) viscosity.

The release characteristics of a tamsulosin salt may also be modified by a suitable hydrophobic treatment thereof before using it as a material in a direct compression or dry granulation into a tablet, e.g. by microensapsulation into a wax shell. Hydrogenated vegetable oils such as hydrogenated castor oil are examples of a suitable wax. The so produced tamsulosin microgranules (with a diameter less than 0.1 mm) may be formulated by any dry process. In one embodiment, the formulated tablet may comprise a disintegrant which allows the tablet to decompose in the stomach environment to liberate the plurality of microgranules with microencapsulated tamsulosin. The microgranule then releases tamsulosin in a desired rate.

In yet another embodiment, two types of release control agents may be combined together to induce both time-dependent and pH-dependent control of the release of tamsulosin. Use of agents that release the active substance independently on environmental pH prevents a dose dumping after the tablet surface comes into contact with the body fluid, while agents releasing the active substance pH-dependently allow to focus the release of a main portion of the active component into desired part of gastrointestinal tract. An example of the polymer that releases substances independently of the pH is Eudragit RS PO, Methocel K4 MP, Carbopol 971P NF or Kollidon SR, whilst Eudragit L 100-55 is the polymer releasing the active substances dependently on pH. Both Eudragit types are powders and they do not have to be suspended in a liquid vehicle before incorporation into the tablet.

As the tablets of the invention are formulated by a tabletting process in which water is absent, a selection among the above basic inert excipients should be thus made, selecting only such ingredients which have suitable properties for use in a dry tabletting process and excluding the others. For instance, the inert carrier for use in compaction method should have good binding properties but, contrary to the tamsulosin compositions of prior art, the release control agent is not required to exhibit properties of a binder, though such is not excluded.

Two general excipient compositions are useful in the tablets of the present invention. One comprises microcrystalline cellulose as a matrix polymer with a release modifying agent such as one or more acrylate polymers; i.e. Eudragits^{TM}. The composition further preferably comprises a lubricant such as magnesium stearate. The other general composition comprises a calcium phosphate such as dicalcium phosphate anhydrate, and HPMC. This embodiment preferably additionally contains a carbohydrate such as lactose anhydrate. A lubricant such as magnesium stearate is also preferably included. The relative amounts are not particularly limited, but it is preferred that these two or three excipients (cellulose, calcium phosphate and optionally lactose) comprise the majority of the excipients, such as 75% or more. Specifically, the following amounts are preferred: 25 to 40% HPMC, 25-40% calcium phosphate, and 25 to 40 % lactose. More preferably the tablet contains about 30 to 40 % HPMC. Substantially equal amounts of HPMC, calcium phosphate and lactose, i.e. each around 30 to 35 % for a total of 90 to 99.9% is a particularly preferred embodiment. Additional excipients including the lubricant, etc. may also be present. This second general composition is preferred from the standpoint of performance reproducibility.

The tablets may be coated such as with an enteric coat or simply for color or stability reasons.

In general, the tablets may provide for immediate release, i.e. within 1-30 minutes after ingestion, for slow or sustained release, i.e. a release within several hours, or for delayed release, i.e. a release after certain latent period. The desired release rate may vary in dependence on the intended actual use, therapeutic purpose, and therapeutic amount or frequency of administration. Preferably however, the tablets of the invention are slow-release tablets essentially bioequivalent to the marketed capsule product. In this regard the tablets of the present invention are preferably 'once daily' tablets meaning that a normal or typical dosing regime for a patient would be to take a single tablet once a day. The preferred release rate is further specified hereinafter.

The tablet made by the process of the invention is preferably a monolithic tablet, i.e. a tablet which does not disintegrate after ingestion to form a plurality of smaller particles from which the active component is finally released. Instead, the product erodes in the body during release of the active substance. Thus, in a monolithic tablet embodiment, none of the excipients used in the manufacturing process of the invention should serve as a disintegrant. More preferably the tablet is monolithic slow release tablet.

Accordingly, the present invention provides a novel pharmaceutical dosage form for oral administration of tamsulosin to a patient in need thereof. In particular, it provides a tablet, particularly a slow release tablet, with tamsulosin and/or its salt (especially hydrochloride) uniformly dispersed in a tablet matrix, which is formulated by a process in which water is absent.

Advantageously, the tablets of the invention should exhibit the following preferred release rate in dissolution test made in vitro:
- In dissolution of the dosage form in phosphate buffer of pH 6.8, by a basket method at 100 rpm, the following release profile is obtained:
   15-35 % in 30 minutes
   40-75 % in 2 hours
   70-100 % in 5 hours

The same preferred release profile should be obtained by an alternative paddle method at 50 rpm.

For therapeutic purposes, bioabsorption of tamsulosin in body fluids should preferably proceed in the small intestines. Accordingly, the tablets of the invention may also be protected by a suitable gastro-resistant coating which delays the onset of release of the active component from the tablet matrix during the passage thereof in the stomach. Examples of such suitable material for gastro-resistant coating are cellulose acetate phthalate (CAP) (Aquacoat^{TM}), co-processed polyvinyl acetate phthalate (Suretetic™), cellulose acetate trimellitate (CAT), Eudragit-type polymers (methacrylic acid copolymers, methacrylic acid copolymer latex, copolymers of methacrylic acid and (meth)acrylic acid esters), hydropropyl methyl cellulose acetate succinate (HPMCAS), carboxymethyl ethylcellulose (CMEC), polyvinyl acetate phthalate (PVAP).

The release property of the coating may be tested also by a dissolution test of the produced coated tablets. The preferred properties of a coated tablet are, e.g.:
- In dissolution of the dosage form in SGF buffer (simulated gastric fluid), by a Ph.Eur. basket method and at 100 rpm, a maximum of 10 % of tamsulosin is released.
- In the buffer of pH 6.8, the coated tablets should comply with the same dissolution profile as specified above.

The tablets of the present invention can be used directly as a unit dosage form, with or without coating, or two or more tablets can be combined such as in a capsule to form a unit dose. The unit dosage form contains an effective amount of tamsulosin for treating or ameliorating the disease, symptoms, and/or or conditions associated with BPH, hypertension, or congestive heart failure, generally from 0.01 to 10.0 mg, preferably 0.1 to 1 mg, in terms of the free base. Preferable are unit doses comprising 0.2, 0.4 or 0.8 mg tamsulosin hydrochloride per se. A unit dose is normally taken from 1 to 3 times daily, preferably once a day as mentioned above. In the case of a capsule, a sufficient number of tablets, or more properly mini-tablets, are provided based on the concentration of the tamsulosin active material therein, so as to provide an effective amount.

Taking the usual therapeutic dose of tamsulosin into consideration, a tablet with a total mass of between 10 and 300 mg are preferred. As the therapeutic dose of tamsulosin is relatively low, the overall weight of the tablet is advantageously kept as low as possible. Low overall weight of a tablet increases the relative content of tamsulosin in the tablet and thus improves the content uniformity. Furthermore, a small tablet will have a similar rate of the gastro-intestinal transit as the granulated product; thus, results obtained from in-vitro dissolution tests may better predict the actual bioequivalence with the marketed granulated product. From this aspect, preferred tablet weight within the invention is from 25 to 250 mg, though it is not limited to this range. The most preferred tablet weight is of approx. 100 mg.

Accordingly, the tablets of the invention may be either mini-tablets, whereby - whenever produced in a circular shape- the average diameter thereof is from about 1.5 to about 2.5 mm, or they may be produced as normal tablets, with an average diameter of between 2.5 and 15 mm. Apart of a circular shape, tamsulosin compositions may be compressed into oval, round biconvex, pentagonal circumcircle or other suitable tablet shape.

Tablets of the invention comprising unit dosage amount of tamsulosin may be delivered for immediate use in a suitable package unit comprising advantageously form 5 to 100 tablets. Such package may comprise a blister pack comprising advantageously 10, 14, 20, 28 or 30 tablets, or a plastic or glass container/bottle containing the same amounts of tablets. Any suitable pharmaceutically acceptable package material may be used in production the package unit.

A pharmaceutical tablet is "dry made" for purposes of the present invention if the tamsulosin active material is combined with/dispersed in the excipient(s) without the use of a liquid. Generally, a dry made tablet has a different structure under scanning electron microscope examination than a comparable wet made tablet, especially a wet granulation made tablet, and can thus be identified as such without actually witnessing the method of manufacture. In particular, the liquid, typically water, causes changes to the active or excipient(s) or both in terms of particle size, agglomeration, granulate formation, etc. Frequently in a dry made tablet, the tamsulosin active material and at least one excipient will retain their initial form and/or be of a finer size.

The tablets of the present invention are made by a process that does not use the aid of a liquid to evenly mix the tamsulosin active material throughout the excipient(s). Such processes include direct compression and dry granulation of a powdered mixture of the tamsulosin active material and suitable inert excipient(s). The present invention therefore provides a formulation comprising direct compressed tamsulosin admixed with dry excipients in the form of a tablet and a formulation comprising dry granulated and compressed tamsulosin admixed with dry excipients in the form of a tablet.

The homogeneity of the powder blends can be aided by the following techniques:
- progressive mixing
   In an initial mixing step, the tamsulosin active component is efficiently blended with a part of the inert carrier, in the second mixing step this solid pre-blend can be blended with the other solid excipients.
- co-milling
   The active substance is milled together with at least a part of the carrier or release-controlling agent. The milled homogeneous mixture is mixed with the remaining ingredients and subjected to compression into tablets. A more special form of this is micronizing.
- adsorption on a solid carrier
   Prior to the formulation process, a solution or a suspension of the tamsulosin active component in a suitable liquid vehicle is contacted with a solid inert carrier and the liquid is immediately evaporated (e.g. by spray drying); as a result, a free flowing powder or a microgranulate is obtained which is used as the starting tamsulosin active material component for the mixing step of the dry process

In a preferred process, the tamsulosin active material is blended with a pharmaceutically acceptable excipient to form a first blend. This first blend is then subjected to and additional blending step wherein one or more additional excipients are added to the blend to form a precompression blend. The precompression blend is then compressed to form tablets.

Additional processes can be performed between these steps or in conjunction therewith. For example, milling is preferably performed after the first blend is formed and before additional excipient(s) is added. Milling can be done at any time in the process including before the first blending step, after the first blending step, before or during the additional blending step or steps and after the additional blending step(s). The blending itself may be progressive in that a portion of the excipient is added, blended, more excipient added, further blended, etc.

Dry granulation, also known as compaction can also be used in conjunction with this process. Specifically, the first blend or any subsequent blend containing additional excipient can be compacted and then milled to form a second blend. Additional excipient(s) in terms of amount or kind can be added to the second blend and blended therein. Alternatively, the second blend can be the final precompression blend that is ready for compression into tablets.

A direct compression method according to the present invention is described in more detail below. The excipients are sieved through a sieve of a suitable size, e.g. 0.5 mm and, except for the lubricant, are mixed in a suitable mixer, e.g. in a free fall mixer to provide a homogeneous mixture. Finally, the lubricant may be added and the mixture is mixed again. The pre-compression mixture is transferred into a filling chamber of the tabletting equipment and compressed into tablets. The complete procedure takes not more than 1 hour.

A compaction method according to the present invention is described in more detail below. In this case, a dry homogeneous mixture of tamsulosin and inert excipients is passed through a compactor to obtain a granulate or ribbon-like strands which are then milled to a free flowing powder, optionally mixed with other excipients and compressed into a tablet.

Content uniformity of the produced batches of tamsulosin tablets may be tested by a suitable pharmacopieial method, e.g. by the method according to Art. 2.9.6 of European Pharmacopoieia, 3^{rd} Ed. Suitable method for measuring the content of tamsulosin in tablets is high performance liquid chromatography (HPLC). A feature of the present invention is that a batch of tablets according to the present invention made without the aid of a liquid (dry made tablets) exhibit high content uniformity. Specifically, the variation in the amount of tamsulosin active material from the target amount within a single batch is 10 % or less, preferably 5 % or less, more preferably 2.5% or less, based on the target amount of tamsulosin active material. Normally, a few samples are withdrawn at regular intervals and tested to confirm the content uniformity throughout the tabletting process.

Tablets for oral administration of tamsulosin according to the invention may be used in the management of functional treatment of symptomatic benign prostate hypertrophy or hyperplasia (BPH) or other disorders treatable by tamsulosin (the Disorders).

Accordingly, the present invention further provides a method for treating and/or preventing any one or more of the Disorders which comprises administering an effective and/or prophylactic amount to a sufferer in need thereof, of tamsulosin or its pharmaceutically acceptable acid addition salt, particularly tamsulosin hydrochloride, which is formulated into a tablet using a process in which water is absent.

The present invention also provides the use of the tamsulosin tablet composition made by a process in which water is absent, as well as the use of the process for making the tamsulosin tablet composition itself, for the manufacture of a medicament for treating and/or preventing any one or more of the Disorders.

Tablet compositions of the invention may be also used in medical applications in combination with other agents. The combination may be realized in a form of single combination preparation or by separate administration of drugs containing the above agents.

The invention is further illustrated by the following Examples.

### Example 1

Three batches of monolithic tablets were made by progressive mixing and direct compression:
a) Tablet composition

| | | (%) |
|---|---|---|
| Tamsulosin hydrochloride | 0.4 mg | 0.5 |
| Lactose anhydrous | 26.4 mg | 33.0 |
| Dicalcium phosphate anh. | 26.4 mg | 33.0 |
| Hypromelose (HPMC) | 26.4 mg | 33.0 |
| Magnesium stearate | 0.4 mg | 0.5 |
| Total | 80 mg | 100 |

The difference between batches was only in the viscosity value of hypromelose selected:
Batch A contained METHOCEL K4M CR PREMIUM
Batch B contained METHOCEL K15M CR PREMIUM
Batch C contained METHOCEL K100M CR PREMIUM

b) Modus operandi
Tamsulosin hydrochloride was blended (15 min) with anhydrous lactose in a 1:9 ratio (10% of active substance), milled (15 seconds) and blended again (5 min). This preblend was then mixed with the rest of the lactose, dicalcium phosphate and hypromelose (10 min), and finally magnesium stearate was added and mixed (5 min) to form the precompression blend. This progressive mixing system provided tamsulosin homogeneity in the preblend of 97.2-100.4% and in the precompression blend of 88.1-98.6%). Compression was performed in a Korsch EK0 press at standard speed and pressure.
c) Characterization of the produced tablets

| Batch | Weight (mg) | Hardnes s(N) | Height (mm) | Diameter (mm) | Assay (%) |
|---|---|---|---|---|---|
| A | 82.8 | 52 | 2.63 | 5.98 | 93.6 |
| B | 83.5 | 38 | 2.69 | 5.99 | 88.1 |
| C | 81.8 | 52 | 2.66 | 5.99 | 98.6 |

d) Dissolution studies
Dissolution tests were performed on 6 tablets using paddle apparatus at a rotation speed of 50 rpm in 500 ml of phosphate buffer pH 6.8. The drug released was determined by an HPLC method and the standard deviation of all batches was below 3%.

### Example 2

| Ingredients | |
|---|---|
| Rac-tamsulosin HCl | 0.4 mg |
| Lactose anhydrous | 25.6 mg |
| Dicalcium phosphate anhydrous | 25.6 mg |
| Hypromelose (HPMC) | 28.0 mg |
| Magnesium stearate | 0.4 mg |
| Total | 80 mg |

### Manufacturing process

Tamsulosin was blended (Turbula; 15 min) with anhydrous lactose in a 1:9 ratio (10 % of active substance), milled (IKA; 30 seconds) and blended again (Turbula; 5 min). The yield of this process was 99.2 % and tamsulsoin assay was 95.5 %. This pre-blend was then mixed with the rest of the lactose, dicalcium phosphate and hypromelose (Bohle LM40, 5L container). Three progressive mixing times (15, 30 and 45 minutes) were evaluated and homogeneity was excellent in all cases (tamsulosin assay of 101.2 %, 101.7 % and 102.1 %). Finally magnesium stearate was sieved, added and mixed (Bohle LM40; 5 min), obtaining a 99.7 % global yield and tamsulosin assay was 102.5 %. The precompression blend was compressed in either an eccentric press Korsch EK0 or in a rotary press Korsch XL100 (about 15000 tablets). Characterisation of the tablets is presented below:

| Press | Weight (mg) | c.v. (%) | Hardness (N) | S.D. (N) | Height (mm) | Diameter (mm) | Assay (%) |
|---|---|---|---|---|---|---|---|
| Korsch EK0 | 79.8 | 1.00 | 75 | 5 | 2.58 | 6 | -- |
| Korsch XL100 | 80.9 | 1.50 | 85 | 7 | 2.55 | 6 | 103.2* |

### Example 3

The following tablets were made by a progressive mixing scheme similar to the one used in Example 1.

| | | | |
|---|---|---|---|
| | | | |
| Tamsulosin.hcl | 0.4 mg | 0.4 mg | 0.4 mg |
| Lactose anhydrous | 35.2 mg | 30.8 mg | 22.0 mg |
| Dicalcium phosphate anh. | 35.2 mg | 30.8 mg | 22.0 mg |
| Hypromelose | 8.8 mg | 17.6 mg | 35.2 mg |
| Magnesium stearate | 0.4 mg | 0.4 mg | 0.4 mg |
| Total | 80 mg | 80 mg | 80 mg |

### Example 4

### Monolithic tablets made by dry compaction

Two batches of tablets were manufactured by a process that includes compaction, milling, mixing and compression.

Batch 4a. : Compacted monolithic tablet, 6 mm diameter. Contains 33.0% of METHOCEL K15M P.

Batch 4b. Compacted monolithic erodible tablet, 9 mm diameter. Contains 13.2% of METHOCEL K15M P.
a) Tablet composition

| | Batch 4a | Batch 4b |
|---|---|---|
| Tamsulosin hydrochloride | 0.4 mg | 0.4 mg |
| Lactose | 65.6 mg | 215.6 mg |
| Hypromelose (HPMC) | 33.0 mg | 33.0 mg |
| Magnesium stearate | 1.0 mg | 1.0 mg |
| Total | 100 mg | 250 mg |

b) Modus operandi
tamsulosin was blended (15 min), milled (15 seconds) and blended again (5 min), with anhydrous lactose in a 1:9 ratio (10% of active substance). This preblend was then mixed with the rest of lactose, hypromelose and 25% of magnesium stearate (10 min), compacted in Chilsonator (Fitz-Patrick) and milled in Fitz-Mill (Fitz-Patrick), finally the rest of magnesium stearate (75%) was added and then mixed (15 min). Compression was performed in a Korsch EK0 press at standard speed and pressure. Characterization of the tablets is presented in the following table.

| Batch | Weight (mg) | Hardnes s(N) | Height (mm) | Diameter (mm) | Lubrication coefficient |
|---|---|---|---|---|---|
| 4a | 103.3 | 31 | 3.42 | 6 | 98.4 |
| 4b | 251.6 | 42 | 3.71 | 9 | 100 |

### Example 5

### Tamsulosin monolithic slow-release tablets made by direct compression

Charge:

| | |
|---|---|
| Tamsulosin hydrochloride | 4.0 g |
| Eudragit RS PO | 200.4 g |
| Eudragit L 100 | 50.1 mg |
| Microcrystalline cellulose | 736.1 g |
| Magnesium stearate | 10.1 g |

All the ingredients except magnesium stearate were charged into a free fall mixer and homogenized by mixing for 40 minutes. Magnesium stearate was added and the mixing continued for additional 5 minutes. A tablet of the weight of 100 mg containing 0.4 mg of tamsulosin hydrochloride was prepared from the mixture by direct compression.

### Example 6.

### Monolithic coated slow release tablets made by progressive mixing and direct compression

Charge:

| | |
|---|---|
| Tamsulosin.HCl | 18 g |
| Eudragit RS PO | 675 g |
| Eudragit L 100 | 225 g |
| MCC 102 | 3527 g |
| Magnesium stearate | 45 g |

Tamsulosin was mixed with 360 g of microcrystalline cellulose for 30 min in a free-fall mixer, 1800 g of microcrystalline cellulose was added and mixing continued for 30 min. Afterwards the remainder of the microcrystalline cellulose (1377 g) and the Eudragits were added. Mixing continued for 30 minutes in free fall mixer. Magnesium stearate was added, and mixed with the other excipients for 5 minutes. Tablets were prepared with an eccentric press. Target weight 100 mg, hardness 100 N. 2.5 kg of tablets were coated with 5 % coating. The 5 % coating was applied in a Bohle LC 5, perforated drum in 60 minutes time.

Coating formulation:

| | |
|---|---|
| Eudragit L 30 D-55 | 416.5 g |
| triethylcitrate | 12.5 g |
| talc | 37.5 g |
| water | 450.0 g |

## Claims

1. A pharmaceutical tablet obtainable by a process without the use of a liquid comprising 0.1 to 1.5 % tamsulosin active material and at least one pharmaceutically acceptable excipient.

2. The pharmaceutical tablet according to claim 1, wherein said tamsulosin active material is tamsulosin free base, a tamsulosin Pharmaceutically acceptable salt or a mixture of two or more of any of the preceding.

3. The pharmaceutical tablet according to claim 1 or 2, wherein said tamsulosin active material is in the form of particles having a distribution of sizes wherein at least 90% have a pacticle size of 100 µm (microns) or less.

4. The pharmaceutical tablet according to claim 3, wherein said tamsulosin active material a particle size distribution wherein at least 90% of said particles have a size of 50 µm (microns) or less.

5. The pharmaceutical tablet according to claim 4, wherein said tamsulosin active material has a particle size distribution wherein at least 90 % of said particles have a size of 20 µm (microns) or less.

6. The pharmaceutical tablet according to claim 4, wherein said tamsulosin active material has a particle size distribution wherein at least 90 % of said particles have a size of 10 µm (microns) or less.

7. The pharmaceutical tablet according to claim 1-6, wherein said tamsulosin active material is contained in an amount of 0.2 to 1.0 %.

8. The pharmaceutical tablet according to claim 7, wherein said tamsulosin active material is contained in an amount of 0.2 to 0.8 %.

9. The pharmaceutical tablet according to claim 7, wherein said tamsulosin active material is contained in an amount of 0.3 to 0.6 %.

10. The pharmaceutical tablet according to claim 1-9, wherein said tamsulosin active material is contained in an amount equivalent to 0.3mg to 12 mg of tamsulosin free base.

11. The pharmaceutical tablet according to claim 1-10, wherein said tamsulosin active material is tamsulosin hydrochloride and said material is contained in an amount of 0.4mg +/- 0.04.

12. The pharmaceutical tablet according to claim 1-11, which comprises at least one pharmaceutically acceptable excipient selected from the group comprising of a polymer, a carbohydrate, and a compressible diluent.

13. The pharmaceutical tablet according to claim 12, which comprises a polymer selected from the group comprising of acrylate polymers and celluloses.

14. the pharmaceutical tablet according to claim 12 or 13, wherein said polymer is selected from HPMC and microcrystalline cellulose.

15. The pharmaceutical tablet according to claim 12-14, which comprises lactose.

16. The pharmaceutical tablet according to claim 12-15, which comprises a calcium phosphate.

17. The pharmaceutical tablet according to claim 12-16, which comprises lactose, HPMC, a calcium phosphate, and magnesium stearate.

18. The pharmaceutical tablet of claim 1-17, which is a monolithic tablet, preferably a slow-release monolithic tablet.

19. The pharmaceutical tablet according to claim 1-18, which further comprises an outer coating.

20. The pharmaceutical tablet according to claim 1-19,wherein said tablet is a once daily extended release tablet.

21. A batch of pharmaceutical tablets, comprising a plurality of dry made tablets containing between 0.1 and 1.5% of a tamsulosin active material and at least one pharmaceutically acceptable excipient, wherein variation in the amount of tamsulosin active material from the target amount is not more than +/-10%.

22. The batch according to claim 21, wherein said variation in tamsulosin active material is not more than +/-5%.

23. The batch according to claim 22, wherein said variation in tamsulosin active material is not more than +/-2;5%.

24. Use of tamsulosine for the preparation of a unit dosage form for treating or ameliorating the conditions of benign prostatic hyperplasia wherein the unit dosage form comprises an effective amount of one or more tablets according to claim 1.20.

25. The use according to claim 24, wherein the unit dosage form comprises two or more of said tablets in a capsule.

26. A process for making tamsulosin tablets which comprises forming a tablet without the aid of a liquid wherein said tablet contains 0.1 to 1.5 % tamsulosin active material and at least one pharmaceutically acceptable excipient.

27. The process according to claim 26, which comprises:
blending a tamsulosin active material with a pharmaceutically acceptable excipient to form a first blend;
additionally blending said first blend with one or more additional excipients in one or more steps to form a precompression blend; and
compressing said precompression blend into tablets.

28. The process according to claim 26 or 27, wherein said first blend is subjected to milling before additional excipients are blended therewith.

29. The process according to claim 26-28, wherein said additional blending further comprises forming a compact of the blended material after at least one additional excipient has been blended with said first blend and milling said compact to form a second blend.

30. The process according to claim 29, wherein said second blend is said precompression blend.

31. The process according to claim 29 or 30, wherein said second blend is further blended with additional excipient to form said precompression blend.

32. The process according to claim 26-31, wherein said tamsulosin active material is in the form of particles having a distribution of sizes wherein at least 90% have a particle size of 100 microns or less.

33. A tamsulosin tablet obtainable by the process of claim 26-32.

## Revendications

1. Comprimé pharmaceutique obtenu selon un procédé sans l'utilisation d'un liquide comprenant 0,1 à 1,5% d'ingrédient actif tamsulosine et au moins un excipient acceptable du point de vue pharmaceutique,

2. Comprimé pharmaceutique selon la revendication 1, dans lequel ledit ingrédient actif est la tamsulosine base libre, un sel de tamsulosine acceptable du point de vue pharmaceutique ou un mélange de deux ou plusieurs parmi l'un quelconque des composés précédents.

3. Comprimé pharmaceutique selon la revendication 1 ou 2, dans lequel ledit ingrédient actif tamsulosine est sous forme de particules présentant une distribution de tailles dans laquelle au moins 90% possèdent une taille de particule de 100 µm (microns) ou moins.

4. Comprimé pharmaceutique selon la revendication 3, dans lequel ledit ingrédient actif tamsulosine présente une distribution de taille de particule dans laquelle au moins 90% desdites particules possèdent une taille de 50 µm (microns) ou moins.

5. Comprimé pharmaceutique selon la revendication 4, dans lequel ledit ingrédient actif tamsulosine présente une distribution de taille de particule dans laquelle au moins 90% desdites particules possèdent une taille de 20 µm (microns) ou moins.

6. Comprimé pharmaceutique selon la revendication 4, dans lequel ledit ingrédient actif tamsulosine présente une distribution de taille de particule dans laquelle au moins 90% desdites particules possèdent une taille de 10 µm (microns) ou moins.

7. Comprimé pharmaceutique selon les revendications 1 à 6, dans lequel ledit ingrédient actif tamsulosine est contenu en une quantité de 0,2 à 1,0%.

8. Comprimé pharmaceutique selon la revendication 7, dans lequel ledit ingrédient actif tamsulosine est contenu en une quantité de 0,2 à 0,8%.

9. Comprimé pharmaceutique selon la revendication 7, dans lequel ledit ingrédient actif tamsulosine est contenu en une quantité de 0,3 à 0,6%.

10. Comprimé pharmaceutique selon les revendications 1 à 9, dans lequel ledit ingrédient actif tamsulosine est contenu en une quantité équivalente à 0,3 mg à 1,2 mg de tamsulosine base libre.

11. Comprimé pharmaceutique selon les revendications 1 à 10, dans lequel ledit ingrédient actif tamsulosine est le chlorhydrate de tamsulosine et ledit ingrédient est contenu en une quantité de 0,4 mg ± 0,04.

12. Comprimé pharmaceutique selon les revendications 1 à 11, qui comprend au moins un excipient acceptable du point de vue pharmaceutique choisi dans le groupe comprenant un polymère, un carbohydrate, et un diluant compressible.

13. Comprimé pharmaceutique selon la revendication 12, qui comprend un polymère choisi dans le groupe comprenant des polymères acrylate et des celluloses.

14. Comprimé pharmaceutique selon la revendication 12 ou 13, dans lequel ledit polymère est choisi parmi HPMC et cellulose microcristalline.

15. Comprimé pharmaceutique selon les revendications 12 à 14, qui comprend du lactose.

16. Comprimé pharmaceutique selon les revendications 12 à 15, qui comprend du phosphate de calcium.

17. Comprimé pharmaceutique selon les revendications 12 à 16, qui comprend du lactose, du HPMC, du phosphate de calcium, et du stéarate de magnésium.

18. Comprimé pharmaceutique selon les revendications 1 à 17, qui est un comprimé monolithique, de préférence un comprimé monolithique à libération lente.

19. Comprimé pharmaceutique selon les revendications 1 à 18, qui comprend de plus un revêtement externe.

20. Comprimé pharmaceutique selon les revendications 1 à 19, dans lequel ledit comprimé est un comprimé à libération prolongée pour une prise journalière.

21. Lot de comprimés pharmaceutiques, comprenant un grand nombre de comprimés fabriqués à sec contenant entre 0,1 et 1,5% d'un ingrédient actif tamsulosine et au moins un excipient acceptable du point de vue pharmaceutique, dans lequel la variation de la quantité d'ingrédient actif tamsulosine par rapport à la quantité cible n'est pas supérieure à ± 10%.

22. Lot selon la revendication 21, dans lequel ladite variation en ingrédient actif tamsulosine n'est pas supérieure à ± 5%.

23. Lot selon la revendication 22, dans lequel ladite variation en ingrédient actif tamsulosine n'est pas supérieure à ± 2,5%.

24. Utilisation de tamsulosine pour la préparation d'une forme de dosage unitaire pour le traitement ou l'amélioration d'états d'hyperplasie prostatique bénigne dans laquelle la forme de dosage unitaire comprend une quantité efficace d'un ou plusieurs comprimés selon les revendications 1 à 20.

25. Utilisation selon la revendication 24, dans laquelle la forme de dosage unitaire comprend deux ou plusieurs desdits comprimés dans une capsule.

26. Procédé de fabrication de comprimés de tamsulosine qui comprend la formation d'un comprimé sans l'aide d'un liquide dans lequel ledit comprimé contient 0,1 à 1,5% d'ingrédient actif tamsulosine et au moins un excipient acceptable du point de vue pharmaceutique.

27. Procédé selon la revendication 26, qui comprend :
le mélange d'un ingrédient actif tamsulosine avec un excipient acceptable du point de vue pharmaceutique pour former un premier mélange ;
le mélange supplémentaire dudit premier mélange avec un ou plusieurs excipients supplémentaires en une ou plusieurs étapes pour former un mélange de précompression ; et
la compression dudit mélange de précompression en comprimés.

28. Procédé selon la revendication 26 ou 27, dans lequel ledit premier mélange est soumis à un broyage avant que des excipients supplémentaires ne soient mélangés à celui-ci.

29. Procédé selon les revendications 26 à 28, dans lequel ledit mélange supplémentaire comprend de plus la formation d'une masse compacte du matériau mélangé après qu'au moins un excipient supplémentaire ait été mélangé avec ledit premier mélange et le broyage de ladite masse compacte pour former un second mélange.

30. Procédé selon la revendication 29, dans lequel ledit second mélange est dit mélange de précompression.

31. Procédé selon la revendication 29 ou 30, dans lequel ledit second mélange est de plus mélangé avec un excipient supplémentaire pour former ledit mélange de précompression.

32. Procédé selon les revendications 26 à 31, dans lequel ledit ingrédient actif tamsulosine est sous forme de particules possédant une distribution de tailles dans laquelle au moins 90% possèdent une taille de particule de 100 microns ou moins.

33. Comprimé de tamsulosine susceptible d'être obtenu selon le procédé des revendications 26 à 32.

## Patentansprüche

1. Pharmazeutische Tablette, erhältlich durch ein Verfahren ohne Verwendung einer Flüssigkeit, umfassend 0,1 bis 1,5% Tamsulosin-Wirkstoff und wenigstens einen pharmazeutisch unbedenklichen Hilfsstoff.

2. Pharmazeutische Tablette gemäß Anspruch 1, worin der Tamsulosin-Wirkstoff eine freie Tamsulosin-Base, ein pharmazeutisch unbedenkliches Tamsulosin-Salz oder eine Mischung aus zwei oder mehr der genannten Substanzen ist.

3. Pharmazeutische Tablette gemäß Anspruch 1 oder 2, worin der Tamsulosin-Wirkstoff die Form von Partikeln mit einer Größenverteilung aufweist, wobei wenigstens 90% eine Teilchengröße von 100 µm (Mikron) oder weniger aufweist.

4. Pharmazeutische Tablette gemäß Anspruch 3, worin der Tamsulosin-Wirkstoff eine Teilchengrößenverteilung aufweist, bei der wenigstens 90% der Teilchen eine Größe von 50 µm (Mikron) oder weniger aufweist.

5. Pharmazeutische Tablette gemäß Anspruch 4, worin der Tamsulosin-Wirkstoff eine Teilchengrößenverteilung aufweist, bei der wenigstens 90% der Teilchen eine Größe von 20 µm (Mikron) oder weniger aufweist.

6. Pharmazeutische Tablette gemäß Anspruch 4, worin der Tamsulosin-Wirkstoff eine Teilchengrößenverteilung aufweist, bei der wenigstens 90% der Teilchen eine Größe von 10 µm (Mikron) oder weniger aufweist.

7. Pharmazeutische Tablette gemäß einem der Ansprüche 1-6, worin der Tamsulosin-Wirkstoff in einer Menge von 0,2 bis 1,0% enthalten ist.

8. Pharmazeutische Tablette gemäß Anspruch 7, worin der Tamsulosin-Wirkstoff in einer Menge von 0,2 bis 0,8% enthalten ist.

9. Pharmazeutische Tablette gemäß Anspruch 7, worin der Tamsulosin-Wirkstoff in einer Menge von 0,3 bis 0,6% enthalten ist.

10. Pharmazeutische Tablette gemäß einem der Ansprüche 1 - 9, worin der Tamsulosin-Wirkstoff in einer Menge enthalten ist, die äquivalent zu 0,3 mg bis 1,2 mg freie Tamsulosin-Base ist.

11. Pharmazeutische Tablette gemäß einem der Ansprüche 1-10, worin der Tamsulosin-Wirkstoff Tamsulosin-Hydrochlorid ist und in einer Menge von 0,4 mg +/- 0,04 enthalten ist.

12. Pharmazeutische Tablette gemäß einem der Ansprüche 1 - 11, welche wenigstens einen pharmazeutisch unbedenklichen Hilfsstoff, ausgewählt aus der Gruppe umfassend ein Polymer, ein Kohlenhydrat und ein komprimierbares Verdünnungsmittel, umfasst.

13. Pharmazeutische Tablette gemäß Anspruch 12, welche ein Polymer, ausgewählt aus der Gruppe umfassend Acrylatpolymere und Cellulosen, umfasst.

14. Pharmazeutische Tablette gemäß einem der Ansprüche 12 oder 13, worin das Polymer ausgewählt ist aus HPMC und mikrokristalliner Cellulose.

15. Pharmazeutische Tablette gemäß einem der Ansprüche 12-14, welche Lactose umfasst.

16. Pharmazeutische Tablette gemäß einem der Ansprüche 12-15, welche ein Calciumphosphat umfasst.

17. Pharmazeutische Tablette gemäß einem der Ansprüche 12-16, welche Lactose, HPMC, ein Calciumphosphat und Magnesiumstearat umfasst.

18. Pharmazeutische Tablette gemäß einem der Ansprüche 1-17, welche eine monolithische, vorzugsweise eine monolithische Tablette mit langsamer Freisetzung ist.

19. Pharmazeutische Tablette gemäß einem der Ansprüche 1-18, welche weiterhin eine äußere Beschichtung umfasst.

20. Pharmazeutische Tablette gemäß einem der Ansprüche 1 - 19, wobei sie eine Tablette zur täglich einmaligen Verabreichung mit verlängerter Feisetzung ist.

21. Eine Charge pharmazeutischer Tabletten, umfassend eine Vielzahl von trocken hergestellten Tabletten, enthaltend zwischen 0,1 und 1,5% eines Tamsulosin-Wirkstoffs und wenigstens einen pharmazeutisch unbedenklichen Hilfsstoff, wobei die Menge an Tamsulosin-Wirkstoff von der Zielmenge um nicht mehr als +/-10% abweicht.

22. Charge gemäß Anspruch 21, wobei die Abweichung in der Menge an Tamsulosin-Wirkstoff nicht größer als +/- 5% ist.

23. Charge gemäß Anspruch 22, wobei die Abweichung in der Menge an Tamsulosin-Wirkstoff nicht größer als +/- 2,5% ist.

24. Verwendung von Tamsulosin für die Herstellung einer Einzel-Darreichungsform zur Behandlung oder Verbesserung der Befindlichkeiten bei benigner Prostatahyperplasie, wobei die Einzel-Darreichungsform eine wirksame Menge einer oder mehrerer Tabletten gemäß einem der Ansprüche 1-20 umfasst.

25. Verwendung nach Anspruch 24, wobei die Einzel-Darreichungsform zwei oder mehrere Tabletten in einer Kapsel umfasst.

26. Verfahren zur Herstellung von Tamsulosin-Tabletten, worin eine Tablette ohne Zuhilfenahme von Flüssigkeit geformt wird, wobei die Tablette 0,1 bis 1,5% Tamsulosin-Wirkstoff und wenigstens einen pharmazeutisch unbedenklichen Hilfsstoff umfasst.

27. Verfahren nach Anspruch 26, umfassend
Mischen eines Tamsulosin-Wirkstoffs mit einem pharmazeutich unbedenklichen Hilfsstoff zur Herstellung einer ersten Mischung;
weiteres Mischen der ersten Mischung mit einem oder mehreren weiteren Hilfsstoffen in einem oder mehreren Schritten zur Herstellung einer vorgepressten Mischung; und
Pressen der vorgepressten Mischung in Tablettenform.

28. Verfahren gemäß einem der Ansprüche 26 oder 27, worin die erste Mischung vor der Mischung mit weiteren Hilfsstoffen einer Zerkleinerung unterzogen wird.

29. Verfahren gemäß einem der Ansprüche 26 - 28, worin das weitere Mischen weiter umfasst, dass aus dem gemischten Material ein Presskörper gebildet wird, nachdem wenigstens ein weiterer Hilfsstoff mit der ersten Mischung vermischt wurde, und dass der Presskörper zur Herstellung einer zweiten Mischung zerkleinert wird.

30. Verfahren gemäß Anspruch 29, worin die zweite Mischung die vorgepresste Mischung ist.

31. Verfahren gemäß einem der Ansprüche 29 oder 30, worin die zweite Mischung weiter mit einem weiteren Hilfsstoff gemischt wird, um die vorgepresste Mischung herzustellen.

32. Verfahren gemäß einem der Ansprüche 26-31, worin der Tamsulosin-Wirkstoff die Form von Partikeln mit einer Größenverteilung aufweist, wobei wenigstens 90% eine Teilchengröße von 100 Mikron oder weniger aufweist.

33. Tamsulosin-Tablette, erhältlich nach dem Verfahren gemäß einem der Ansprüche 26 - 32.
